# EUROPEAN PATENT SPECIFICATION

(11) **EP 2 412 311 B1**
(45) Date of publication and mention of the grant of the patent: **03.12.2014**
(21) Application number: 11008539.6
(22) Date of filing: 11.04.2007
(51) Int. Cl.: A61B 5/1459, A61B 5/145, A61J 15/00, G01N 21/00, G01N 21/77

(54) **Feeding tube position confirmation device**
Positionsbestimmung für Magensonde
Dispositif pour confirmer la position du tube d'alimentatión

(30) Priority: 12.04.2006 GB 0607326
(43) Date of publication of application: 01.02.2012
(62) Divisional of application: 07732385.5
(73) Proprietor: Thomas, Sean Julian, Greasby,Wirral, Merseyside CH49 1SP (GB)
(72) Inventor: Thomas, Sean Julian, Greasby,Wirral, Merseyside CH49 1SP (GB)

(56) References cited:
- EP-A- 0 481 740
- US-A- 4 200 110
- US-A- 4 381 011
- US-A- 4 577 109
- US-A- 5 085 216
- US-A- 5 105 812

## Description

The present invention relates to medical feeding tubes, such as, for example, nasogastric and nasointestinal feeding tubes, and more particularly to facilitate placement and/or avoid misplacement of the portion of the tube through which the fluid exits.

In healthcare, human or animal patients may be incapable of feeding themselves by conventional means. In such circumstances it is necessary to deliver nutrients into the stomach or small intestine by way of a feeding tube. This is generally carried out by passing a tube through the patient's nasal passage and into the stomach or the small intestine by way of the gastrointestinal tract. The distal end of such feeding tubes comprise one or more fluid output apertures, which act to deliver fluid nutrients to predetermined locations such as, for example, the stomach or small intestine. Correct positioning of the fluid output apertures within the stomach or small intestine is essential for the safety of the patient. For example, misdirection of the feeding tube upon insertion via the nasal cavity such that the leading end of the feeding tube is directed towards the lungs may occur, particularly with patients who have an inhibited cough or gag reflex such as, for example, the critically ill and premature babies. Such misplacement of the fluid output apertures may lead to serious pleuropulmonary complications such as, for example, pneumonia, abscess and empyema.

Also, in certain circumstances it is beneficial for the patient if certain fluid nutrients are delivered to specific parts of the digestive system such as, for example, specifically to the stomach and/or specifically to the small intestine. Again, correct placement of the fluid outlet apertures is essential.

Generally, health practitioners currently approximate the position of the fluid output apertures before confirming the correct position. A commonly used method for confirming placement of the fluid output apertures is to connect a syringe to the proximate end of a pre-placed feeding tube and aspirate some fluid from the region around the fluid output apertures. The pH of the aspirated fluid is then measured to determine whether, for example, the pH of the fluid corresponds with the pH of gastric fluid from the stomach thereby confirming placement of the fluid output apertures in the stomach. However, it is known for the aspirated gastric fluid to become contaminated as it is transferred from the syringe leading to false readings. Furthermore, significant care has to be taken not to aspirate too much fluid. Also this method can be unpleasant for the patient as it can tend to cause reflux and vomiting which can lead to further complications. Furthermore, it is often necessary and good practice to additionally confirm correct placement of the fluid outlet apertures using radiography whereby the outer surface of the tube has a plurality of spaced apart radiopaque markings which are visible under x-ray, However, although use of radiography provides positive confirmation of correct placement of a tube it is disadvantaged In that it is relatively expensive, as it requires a radiographer, x-ray equipment and also a doctor to confirm correct placement. Furthermore, this method is further disadvantaged in that the patient, who may be critically ill, may also have to be transferred to a radiology department and is also exposed to x-ray.

Patent document number US 4,381,011 discloses a system and method for feeding of fluid into a preselected portion of the gastro intestinal tract of a patient. The system comprises a tube with a pH measuring device positioned thereon, a monitoring device, capable of processing pH signals to determine the position of the tube, and a fluid feed control. Initial positioning of the tube and subsequent monitoring of the position of the tube is accomplished by receiving and processing pH signals from the pH measuring device positioned proximate the distal end of the tube and connected to the monitoring device. The tube may be selectively positioned in a preselected portion of the digestive system by monitoring the pH, which the pH measuring device is measuring, and comparing those measurements with known values of pH for specific portions of the digestive system. However, this system is disadvantaged in that It is relatively expensive, needing pH measuring devices and monitors, and requires a power supply.

Patent document number US 5,085,216 describes a feeding tube assembly for nasogastric and nasointestinal feeding comprising a pH indicator carried by a stiffener used for inserting the feeding tube into a patient. After insertion of the leading end of the feeding tube into an approximated desired position the pH indicator is withdrawn and examined for a pH corresponding to that of the stomach thereby indicating that the end of the tube is positioned in the stomach. However, this feeding tube assembly is disadvantaged in that it is necessary to approximate the correct position of the tube prior to withdrawing the pH indicator to determine whether or not the tube is correctly positioned. If the tube is not correctly positioned in the stomach it is necessary to withdraw the tube from the patient and repeat the whole procedure again using a complete new feeding tube assembly which is undesirably wasteful, time consuming and distressful for the patient Furthermore, the pH indicator may become contaminated as it is withdrawn into the environment external to the body leading to false and unreliable pH readings.

US 5 105 812 discloses a feeding tube confirmation device including all the technical features of the preamble of claim 1.

It is an object of the present invention to provide a device for confirming the position of a feeding tube, which is relatively inexpensive to manufacture, which is disposable, which does not require an electric power source, which is easy to use and which is capable of indicating correct positioning of a tube's fluid outlet apertures during insertion thereof.

According to the present invention there is provided a feeding tube position confirmation device, operable to confirm the position of a predetermined portion of a medical feeding tube in a predetermined portion of a human or animal body, characterised in that the position confirmation device comprises an input optical waveguide and an output optical waveguide and is dimensioned to be insertable into the lumen of a said feeding tube, the position confirmation device further comprising sensor means disposed on at least one of the input and output optical waveguides at a position thereon which corresponds to the predetermined portion of the said feeding tube, the sensor means comprising a colour change indicator operable to change colour relative to the chemical content of the environment proximate thereto, wherein the input optical waveguide is operable to carry light to the sensor means and wherein the colour change indicator is operable to change to a predetermined colour upon detection of a predetermined chemical content, the sensor means thus causing a change in the colour of the input light to provide an output light of a predetermined colour indicative of the position of the said feeding tube, the output light being carried to the proximate end of the position confirmation device, by the output optical waveguide, at which it is viewed by the user to determine the position of the said tube in the human or animal body.

The input optical waveguide and the output optical waveguide may be the same optical waveguide.

The input light may be reflected off the sensor means to provide the output light.

Alternatively, the input light may be transmitted from the input waveguide through the sensor means into the output waveguide.

The sensor means advantageously comprises a CO₂ sensitive colour change indicator.

The CO₂ sensitive colour change indicator is advantageously operable to change colour upon positioning of the predetermined portion of the said tube in the respiratory system of the human or animal body.

Additionally, or alternatively, the sensor means advantageously comprises a pH sensitive colour change indicator.

The pH colour indicator is advantageously operable to change to a predetermined colour upon positioning of a predetermined portion of the said tube in the stomach of the human or animal body.

The change to a predetermined colour advantageously occurs at a pH less than 6.

The pH colour change indicator may be selected from: Congo Red; Bromophenol Blue; Chlorophenol Blue; Bromochlorophenol Blue; Methyl Yellow; Methyl Orange.

The feeding tube position confirmation device may comprise a plurality of sensors.

The feeding tube position confirmation device advantageously has sufficient rigidity such that it is operable as a stiffener to facilitate insertion of the said feeding tube into the human or animal body.

The feeding tube position confirmation device may further comprise an optical detector operable to detect the output light and convert it into an electronic signal indicative of the position of the predetermined position of the feeding tube.

According to a second aspect of the present invention there is provided a medical feeding tube kit comprising a medical feeding tube and at least one feeding tube position confirmation device according to the first aspect of the present invention.

The medical feeding tube kit may further comprise a light source.

Embodiments of the present invention will now be described, by way of example, with reference to the accompanying drawings in which:
Figure 1 is a schematic drawing, in section, of a first embodiment of a tube not part of the present invention;
Figure 2 is a schematic drawing, in section, of a second embodiment of a tube not part of the present invention;
Figure 3 is a schematic drawing, in section, of a third embodiment of a tube not part of the present invention;
Figure 4 is a schematic drawing, in section, of a fourth embodiment of a tube not part of the present invention;
Figure 5 is a schematic drawing, in section, of a fifth embodiment of a tube not part of the present invention;
Figure 6 is a schematic drawing, in section, of a sixth embodiment of a tube not part of the present invention;
Figure 7 is a schematic drawing of a tube kit including positioning means, according to the present invention;
Figure 8 is a drawing showing a tube according to the present invention disposed in a patient;
Figure 9 is a drawing showing the correct and incorrect direction of insertion of a tube, in a patient; and,
Figure 10 is a schematic drawing of tube positioning means according to the present invention.

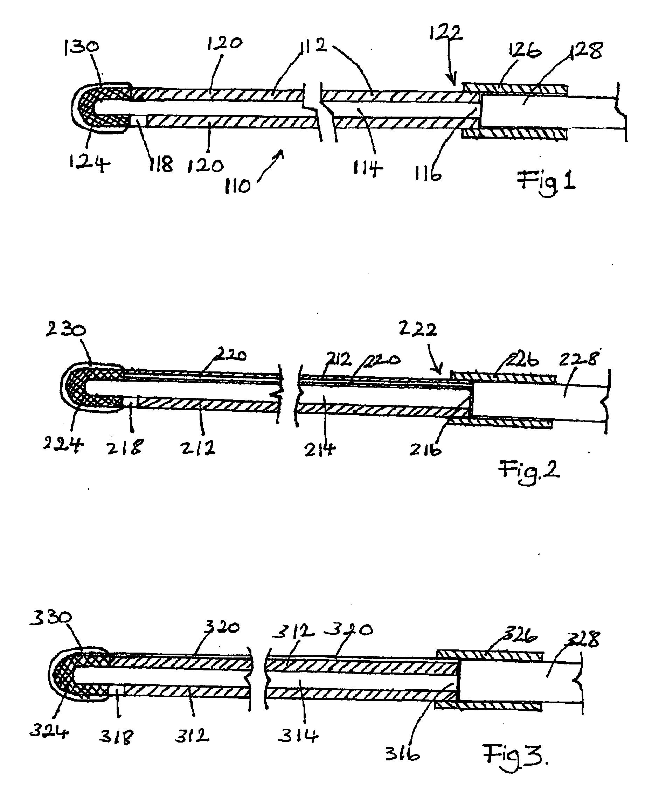

Referring to Figure 1, a first example of a tube 110 comprises a wall 112 defining a lumen 114 through which fluid is delivered between a fluid inlet aperture 116 and a fluid outlet aperture 118. The tube further comprises positioning means having an optical waveguide 120 extending between the proximal end 122 of the tube and sensing means 124.

The wall 112 is formed from a flexible, biocompatibe material, which is optically transmissive such that the wall itself is the optical waveguide.

The sensing means 124 is disposed at, or adjacent, the fluid outlet aperture 118. However, as will become apparent in the description below, the sensing means may be disposed in other portions of the tube provided the position of the fluid outlet aperture can be derived from its position.

The sensor means 124 comprises a pH sensitive indicator which changes colour upon detection of gastric juices of the stomach. Such indicators are known. For example, such an indicator may be selected from the group consisting of Congo Red, Bromophenol Blue, Chlorophenol Blue, Bromochlorophenol Blue, Methyl Yellow and Methyl Orange. However, it will be appreciated that other known pH indicators may be used with equal effect provided they are suitably biocompatible and indicate a change in pH in suitable range.

Alternatively, or additionally, the sensing means may comprise a carbon dioxide (CO2) sensitive indicator, which changes colour upon the detecting CO2 in the environment proximate thereto. Such indicators are known in the art. The indicators may be immobilised within the wall 112 or coated on the outer and/or inner surface of the wall 112, at the desired portion of the tube.

The tube 10 further comprises an adapter 128 suitable to receive an optical source 128. The adapter 126 is also suitably sized to receive an outlet tip of a syringe or outlet from another fluid supply source.

An optical reflector 30 may be used on the distal end 132 of the tube in order to reduce optical losses from the waveguide 120 and to facilitate reflection of light as described later in the description. The optical reflector may be In the form of a reflective coating or cap.

Referring to Figure 2, a second example of a tube 210, according to the present invention, is shown. For clarity, where the features of the first embodiment are also present in the second embodiment the corresponding reference numbers have been used.

The second embodiment of the tube 210 is identical to the first embodiment, of Figure 1, except the wall 212 does not primarily function as the optical waveguide 220. Instead, the optical waveguide 220 is embedded in the wall 212.

Referring to Figure 3, a third example of a tube 310, according to the present invention, is shown. For clarity, where the features of the first embodiment are also present in the third example the corresponding reference numbers have been used.

The third example of the tube 310 is identical the first embodiment, of Figure 1, except the wall 312 does not primarily function as the optical waveguide 320. Instead the optical waveguide 320 is disposed on the outer surface of the wall 312. However, it will be appreciated that the waveguide 320 may alternatively extend along the inner surface of the wall 312.

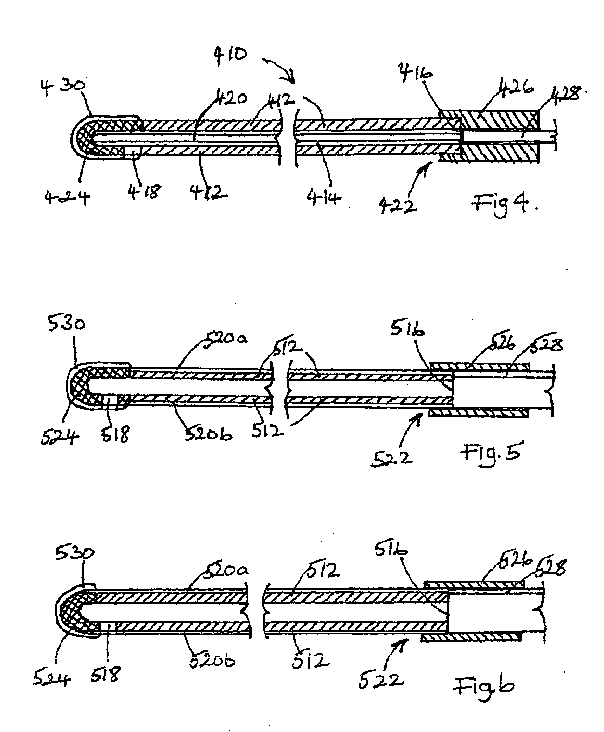

Referring to Figure 4, a fourth example of a tube 410 is shown. For clarity, where the features of the first embodiment are also present in the fourth embodiment the corresponding reference numbers have been used.

The fourth embodiment of the tube 410 is identical to the first embodiment, of Figure 1, except the wall 412 does not primarily function as the waveguide 420. Instead the waveguide 420 is positioned within the lumen 414 and is removable therefrom via the fluid inlet aperture 416.

Referring to Figures 1 to 4, the waveguide (120, 220, 320, 420) is adapted to receive light from the optical source (128, 228, 328, 428), disposed at the proximal end (122, 222, 322, 422) of the tube. The waveguide carries an input optical signal to illuminate the sensor means (124, 224, 324, 424) where the light is reflected back, as an output optical signal, along the waveguide and carried thereby to the proximal end of the tube where it can be viewed and/or recorded. The optical reflector (130, 230, 330, 440) facilitates reflection of the light at the sensing means.

Referring to Figures 5 and 6, a fifth and sixth example of a tube, according to the present invention, is shown. Again, for clarity, where the features of the first embodiment are also present in the fifth and sixth embodiments, corresponding reference numbers have been used.

Figures 5 and 6 show a tube, which is identical to the tube of the first example, of Figure 1, except the wall 512 does not primarily function as the waveguide. Instead, the waveguide 520 comprises an input waveguide 520a, which extends from the proximal end 522 of the tube to the sensing means 524, and an output waveguide 520b, which extends from the sensing means 524 to the proximal end 522 of the tube. Figure 5 shows a tube wherein the waveguides 520a and 520b are disposed within the wall 512, for example, they may be embedded within the wall, whereas Figure 6 shows a tube wherein the waveguides 520 a and 520b are disposed on the external surface of the wall 512. However, it will be appreciated that the waveguides 520a and 520b may alternatively be disposed on the internal surface of the wall 512.

The input waveguide 520a is adapted to receive light from a light source 528 disposed at the proximal end 522 of the tube. The input waveguide 520a carries the input optical signal to the sensing means 524 through which the input optical signal is transmitted. An output optical signal is carried from the sensor means 524 to the proximal end 522 of the tube, by the output waveguide 520b, where it can be viewed and/or recorded by a user.

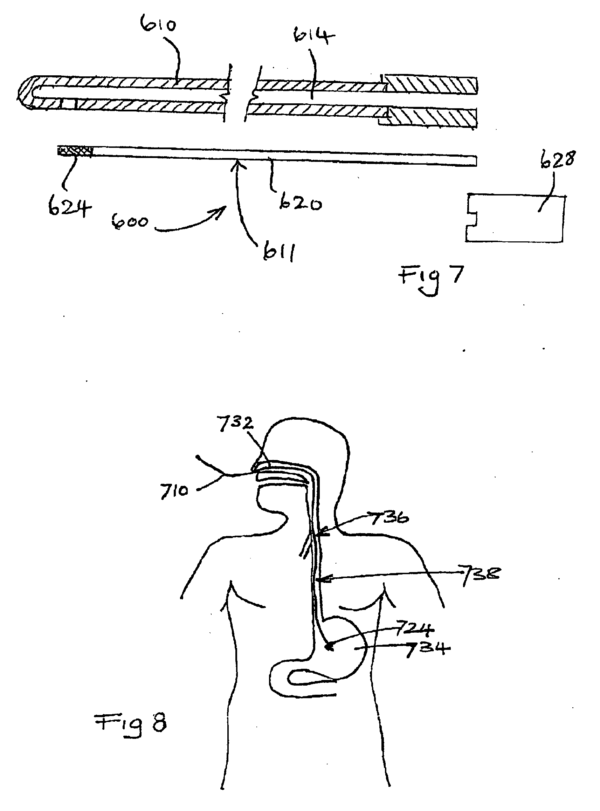

Referring to Figure 7, a nasogastric tube kit 600 comprises a tube 610, of a standard known type, and tube positioning means or a feeding tube position confirmation device 611. The tube positioning means is specifically dimensioned in cross section and length to compliment and correspond to a specific size of nasogastric feeding tube such that it is insertable into the lumen 614 of standard known nasogastric tubes.

The tube positioning means has an optical waveguide 620 and sensor means 624. The optical waveguide 620 may comprise one waveguide which is used for both the input and output optical signal (i.e. wherein light is reflected off the sensor means) or an input waveguide and an output waveguide (i.e. wherein the light is transmitted from the input waveguide through the sensor means and into the output waveguide), as described in relation to the previous example. As for the previous examples the one or more optical waveguides may be formed from one or more optical fibres.

The sensor means 624 comprises a pH sensitive indicator which changes colour upon detection of gastric juices of the stomach, as described above in relation to the previous embodiments. Alternatively, or additionally, the sensing means may comprise a carbon dioxide (CO2) sensitive indicator, which changes colour upon detecting CO2 in the environment proximate thereto. Such indicators are known in the art. The sensor means is disposed on the optical waveguide in a predetermined position such that, in use, it provides positional information in relation to a predetermined portion of the tube.

The kit may further comprise a light source 628 for use with the tube positioning means.

The tube positioning means may be provided as part of a nasogastric tube kit or provided independently for use with known nasogastric tubes.

Referring to Figures 1 to 4, in use, the optical source (128, 228, 328, 428) is positioned relative to the adapter (126, 226, 326, 426) such that light is launched from the optical source (128, 228, 328, 428) into the optical waveguide (120, 220, 320, 420) preferably prior to insertion of the tube. The input optical signal is carried by the waveguide to the sensor means (124, 224, 324, 424), which is illuminated thereby. The input optical signal is reflected back from the sensor means as an output optical signal. The output optical signal is carried to the proximal end (122, 222, 322, 422) of the tube, by the waveguide, where its colour is viewed and/or recorded.

For the examples shown in Figures 5 and 6, the optical source 528 launches an input optical signal into the input waveguide 520a. The input optical signal is carried to the sensor means 524 by the input waveguide 520a and transmits through the sensor means and into the output waveguide 520b as an output optical signal. The output optical signal is carried to the proximal end 522 of the tube, by the output waveguide 520b, where its colour viewed and/or recorded.

Referring again to Figure 7, in use, the tube positioning means 611 is inserted into the tube 610 through the fluid inlet aperture 616, at the proximal end thereof, such that the sensor means 624 is positioned to correspond to the predetermined portion of the tube. The light source 628 is positioned relative to the tube placement means 620 such that it launches light, in the form of an input optical signal, into the waveguide 620 thereof. The input optical signal travels along the waveguide and illuminates the sensor means 624 before being reflected back up the waveguide as an output optical signal. The colour of the output optical signal is viewed and/or recorded at the proximate end of the tube positioning means.

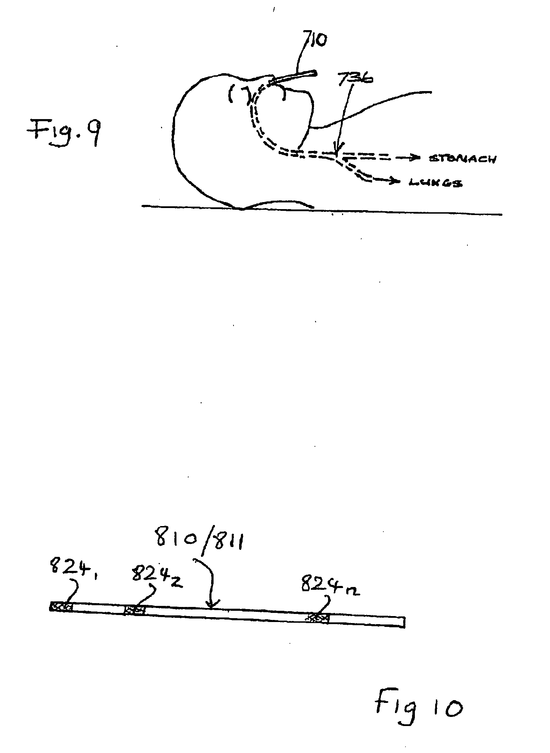

Referring also to Figures 8 and 9, in healthcare a nasogastric tube 710, or nasogastric tube having tube positioning means, according to any one of the embodiments of the present invention, is inserted into the nasal passage 732 of a patient to deliver fluid nutrients and/or medicines to part of the digestive system, for example, the stomach 734. Correct placement of the tube is essential as misplacement can cause serious complications. Placement of the fluid output aperture 718 in the respiratory system can occur through misdirecting the tube at the epiglottis 736, as shown in Figure 9. Furthermore even if the tube is directed towards the digestive system the fluid outlet aperture 718 may still be misplaced in the oesophagus 738 from which fluid may be inhaled into the respiratory system with serious consequences for the patient.

Where the tube, or tube positioning means, comprises sensor means having a CO2 indicator, if the tube is misdirected at the epiglottis 736, such that the sensor means 724 enters the respiratory system, the sensor means 724 changes to a predetermined colour upon detection of CO2. The change in colour of the sensor means 724 changes the properties of the output optical signal, (i.e. a predetermined change in the colour of the output optical signal occurs). The predetermined change in colour of the output optical signal indicates to a person inserting the tube that the tube has been misdirected into the respiratory system and the tube can be redirected into the oesophagus accordingly.

Positioning of the tube continues with the tube being further inserted along the oesophagus 738 towards the stomach 34. Upon entering the stomach the acidity of the gastric fluids change the colour of the sensor means 24 in a predetermined manner. The gastric fluids of the stomach are typically at a pH of about 1.5. However, in practise, gastric fluids may have a pH of up to 5 or 6 and therefore the sensor means should ideally be sensitive to indicate a pH of less than 6. The predetermined change in colour of the sensor means 724 changes the properties of the output optical signal (i.e. a predetermined change in the colour of the output optical signal occurs). For example, where the sensor means 724 comprises Congo Red the gastric fluids change the colour of the sensor means from red to blue. Consequently, upon the sensor means entering the stomach the output optical signal, as viewed by the person inserting the tube, changes from red to blue which indicates, to that person, that the relevant portion of the tube is in the stomach, i.e. the tube has been correctly placed.

In an alternative embodiment the change of colour of the output optical signal may be detected by electronic means, wherein an optical detector detects the optical output signal and converts it into an electronic signal indicative of whether or not a predetermined portion of the tube is in the desired position.

With the tube positioned in its desired position the pH of the stomach may be monitored continuously, intermittently or at least before fluid is passed through the tube.

Referring to Figure 10, tube positioning means 811, according to the present invention, may comprise a plurality of sensor means 824i to 824n> of the type described above, spaced apart from each other along the length of the respective tube or tube positioning means. Each sensor means having one or more optical waveguides associated therewith. Such embodiments of the present invention may be used to confirm positional information of different portions of the tube or tube positioning means.

The tube positioning means, according to the present invention, have been described with reference to exemplary embodiments, each having different arrangements of optical waveguide(s). It will be appreciated that any two of these waveguide arrangements combined are equally applicable to the working of the present invention. Furthermore, it will also be appreciated that the sensing means may be disposed on the tube positioning means, at any position to indicate to a user correct placement of a desired portion of the tube. For example, the sensor means may be disposed at, or adjacent to, the fluid output aperture, as shown in Figures 1 to 7, to indicate that the fluid outlet aperture is in a desired location before fluid is delivered thereto. However, the present invention is equally applicable to a an application wherein the sensor means is spaced apart from a fluid output aperture to indicate to a user that fluid will not be delivered to a particular discrete remote location, i.e. the location in which the sensor means is located.

## Claims

1. A feeding tube position confirmation device (611), operable to confirm the position of a predetermined portion of a medical feeding tube (610) in a predetermined portion of a human or animal body, **characterised in that** the position confirmation device comprises an input optical waveguide (620) and an output optical waveguide (620) and is dimensioned to be insertable into the lumen of a said feeding tube (610), the position confirmation device further comprising sensor means (624) disposed on at least one of the input and output optical waveguides (620) at a position thereon which corresponds to the predetermined portion of the said feeding tube (610), the sensor means (624) comprising a colour change indicator operable to change colour relative to the chemical content of the environment proximate thereto, wherein the input optical waveguide (620) is operable to carry light to the sensor means (624) and wherein the colour change indicator is operable to change to a predetermined colour upon detection of a predetermined chemical content, the sensor means (624) thus causing a change in the colour of the input light to provide an output light of a predetermined colour indicative of the position of the said feeding tube (610), the output light being carried to the proximate end of the position confirmation device, by the output optical waveguide, at which it is viewed by the user to determine the position of the said tube in the human or animal body.

2. A feeding tube position confirmation device as claimed in claim 1, wherein the input optical waveguide and the output optical waveguide are the same optical waveguide.

3. A feeding tube position confirmation device as claimed in claim 1 or 2, wherein the input light is reflected off the sensor means to provide the output light.

4. A feeding tube position confirmation device as claimed in claim 1, wherein the input light is transmitted from the input waveguide through the sensor means into the output waveguide.

5. A feeding tube position confirmation device as claimed in any of the preceding claims, wherein the sensor means comprises a CO₂ sensitive colour change indicator.

6. A feeding tube position confirmation device as claimed in claim 5, wherein the CO₂ sensitive colour change indicator is operable to change colour upon positioning of the predetermined portion of the said tube in the respiratory system of the human or animal body.

7. A feeding tube position confirmation device as claimed in any of the preceding claims, wherein the sensor means comprises a pH sensitive colour change indicator.

8. A feeding tube position confirmation device as claimed in claim 7, wherein the pH colour indicator is operable to change to a predetermined colour upon positioning of a predetermined portion of the said tube in the stomach of the human or animal body.

9. A feeding tube position confirmation device as claimed in claim 7, wherein the change to a predetermined colour occurs at a pH less than 6.

10. A feeding tube position confirmation device as claimed in claim 7 to 9, wherein the pH colour change indicator is selected from: Congo Red; Bromophenol Blue; Chlorophenol Blue; Bromochlorophenol Blue; Methyl Yellow; Methyl Orange.

11. A feeding tube position confirmation device as claimed in any of the preceding claims, comprising a plurality of sensors.

12. A feeding tube position confirmation device as claimed in any of the preceding claims, having sufficient rigidity such that it is operable as a stiffener to facilitate insertion of the said feeding tube into the human or animal body.

13. A feeding tube position confirmation device as claimed in any of the preceding claims further comprising an optical detector operable to detect the output light and convert it into an electronic signal indicative of the position of the predetermined position of the feeding tube.

14. A medical feeding tube kit comprising a medical feeding tube and at least one feeding tube position confirmation device as claimed in claims 1 to 13.

15. A medical feeding tube kit as claimed in claim 14, further comprising a light source.

## Patentansprüche

1. Eine Ernährungssonden-Positionsbestätigungsvorrichtung (611), die betätigbar ist, um die Position eines vorbestimmten Abschnitts einer medizinischen Ernährungssonde (610) in einem vorbestimmten Abschnitt eines Menschen- oder Tierkörpers zu bestätigen, **dadurch gekennzeichnet, dass** die Positionsbestätigungsvorrichtung einen optischen Eingangswellenleiter (620) und einen optischen Ausgangswellenleiter (620) umfasst und so bemessen ist, dass sie in das Lumen einer solchen Ernährungssonde (610) eingeführt werden kann, wobei die Ernährungssonden-Positionsbestätigungsvorrichtung ferner ein Sensormittel (624) umfasst, das mindestens an dem Eingangs- und/oder dem Ausgangswellenleiter (620) an einer Position darauf angeordnet ist, welche dem vorbestimmten Abschnitt der Ernährungssonde (610) entspricht, wobei das Sensormittel (624) einen Farbänderungsindikator umfasst, der betätigbar ist, um sich relativ zum chemischen Anteil der dazu unmittelbaren Umgebung zu ändern, wobei der optische Eingangswellenleiter (620) betätigbar ist, um Licht zu dem Sensormittel (624) zu tragen, und wobei der Farbänderungsindikator betätigbar ist, um sich nach Erfassung eines vorbestimmten chemischen Anteils zu einer vorbestimmten Farbe zu ändern, wobei das Sensormittel (624) somit eine Änderung der Farbe des Eingangslichts verursacht, um ein Ausgangslicht mit einer vorbestimmten Farbe bereitzustellen, welche auf die Position der Ernährungssonde (610) hinweist, wobei das Ausgangslicht zu dem benachbarten Ende der Positionsbestätigungsvorrichtung getragen wird, durch den optischen Ausgangswellenleiter, wo es von dem Benutzer gesehen wird, sodass er die Position der Sonde im Menschen- oder Tierkörper bestimmen kann.

2. Ernährungssonden-Positionsbestätigungsvorrichtung nach Anspruch 1, wobei der optische Eingangswellenleiter und der optische Ausgangswellenleiter derselbe optische Wellenleiter sind.

3. Ernährungssonden-Positionsbestätigungsvorrichtung nach Anspruch 1 oder 2, wobei das Eingangslicht von dem Sensormittel reflektiert wird, um das Ausgangslicht bereitzustellen.

4. Ernährungssonden-Positionsbestätigungsvorrichtung nach Anspruch 1, wobei das Eingangslicht von dem Eingangswellenleiter durch das Sensormittel in den Ausgangswellenleiter geleitet wird.

5. Ernährungssonden-Positionsbestätigungsvorrichtung nach einem der vorhergehenden Ansprüche, wobei das Sensormittel einen CO₂-empfindlichen Farbänderungsindikator umfasst.

6. Ernährungssonden-Positionsbestätigungsvorrichtung nach Anspruch 5, wobei der CO₂-empfindliche Farbänderungsindikator betätigbar ist, um nach Positionierung des vorbestimmten Abschnitts der Sonde im Atmungssystem des Menschen- oder Tierkörpers die Farbe zu ändern.

7. Ernährungssonden-Positionsbestätigungsvorrichtung nach einem der vorergehenden Ansprüche, wobei das Sensormittel einen pH-empfindlichen Farbänderungsindikator umfasst.

8. Ernährungssonden-Positionsbestätigungsvorrichtung nach Anspruch 7, wobei der pH-empfindliche Farbänderungsindikator betätigbar ist, um sich nach Positionierung eines vorbestimmten Abschnitts der Sonde im Magen eines Menschen- oder Tierkörpers zu einer vorbestimmten Farbe zu ändern.

9. Ernährungssonden-Positionsbestätigungsvorrichtung, nach Anspruch 7, wobei die Änderung zu einer bestimmten Farbe bei einem pH-Wert von weniger als 6 geschieht.

10. Ernährungssonden-Positionsbestätigungsvorrichtung nach Anspruch 7 bis 9, wobei der pH-Farbänderungsindikator ausgewählt ist aus; Kongorot; Bromphenolblau; Chlorphenolblau; Bromchlorphenolblau; Methylgelb; Methylorange.

11. Ernährungssonden-Positionsbestätigungsvorrichtung nach einem der vorhergehenden Ansprüche, umfassend eine Vielzahl von Sensoren.

12. Ernährungssonden-Positionsbestätigungsvorrichtung nach einem der vorhergehenden Ansprüche, wobei sie eine ausreichende Steifigkeit aufweist, so dass sie als Aussteifelement betätigbar ist, um das Einführen der Ernährungssonde in den Menschen- oder Tierkörper zu erleichtern.

13. Ernährungssonden-Positionsbestätigungsvorrichtung nach einem der vorhergehenden Ansprüche, ferner umfassend einen optischen Detektor, der betätigbar ist, um das
Ausgangslicht zu detektieren und es in ein elektronisches Signal umzuwandeln, das auf die Position der vorbestimmten Position der Ernährungssonde hinweist.

14. Ein medizinisches Ernährungssonden-Kit, umfassend eine medizinische Ernährungssonde und mindestens eine Ernährungssonden-Positionsbestätigungsvorrichtung nach Ansprüchen 1 bis 13.

15. Medizinisches Ernährungssonden-Kit nach Anspruch 14, ferner umfassend eine Lichtquelle.

## Revendications

1. Un dispositif de confirmation de position de sonde d'alimentation (611), utilisable pour confirmer la position d'une portion prédéterminée d'une sonde d'alimentation médicale (610) dans une portion prédéterminée d'un corps humain ou animal, **caractérisé en ce que** le dispositif de confirmation de position comporte un guide d'onde optique d'entrée (620) et un guide d'onde optique de sortie (620) et est dimensionné pour pouvoir être inséré dans la lumière d'une dite sonde d'alimentation (610), le dispositif de confirmation de position comportant en outre un moyen capteur (624) disposé sur au moins un guide d'onde parmi les guides d'onde optique d'entrée et de sortie (620) au niveau d'une position sur ce dernier qui correspond à la portion prédéterminée de ladite sonde d'alimentation (610), le moyen capteur (624) comportant un indicateur de changement de couleur utilisable pour changer de couleur relativement au contenu chimique de l'environnement proche de celui-ci, dans lequel le guide d'onde optique d'entrée (620) est utilisable pour transporter de la lumière jusqu'au moyen capteur (624) et dans lequel l'indicateur de changement de couleur est utilisable pour changer en une couleur prédéterminée après détection d'un contenu chimique prédéterminée, le moyen capteur (624) entraînant ainsi un changement de la couleur de la lumière d'entrée pour fournir une lumière de sortie d'une couleur prédéterminée indicative de la position de ladite sonde d'alimentation (610), la lumière de sortie étant transportée jusqu'à l'extrémité proche du dispositif de confirmation de position, par le guide d'onde optique de sortie, au niveau de laquelle elle est visualisée par l'utilisateur pour déterminer la position de ladite sonde dans le corps humain ou animal.

2. Un dispositif de confirmation de position de sonde d'alimentation tel que revendiqué dans la revendication 1, dans lequel le guide d'onde optique d'entrée et le guide d'onde optique de sortie sont le même guide d'onde optique.

3. Un dispositif de confirmation de position de sonde d'alimentation tel que revendiqué dans la revendication 1 ou la revendication 2, dans lequel la lumière d'entrée est réfléchie sur le moyen capteur pour fournir la lumière de sortie.

4. Un dispositif de confirmation de position de sonde d'alimentation tel que revendiqué dans la revendication 1, dans lequel la lumière d'entrée est transmise du guide d'onde d'entrée par le biais du moyen capteur jusque dans le guide d'onde de sortie.

5. Un dispositif de confirmation de position de sonde d'alimentation tel que revendiqué dans n'importe lesquelles des revendications précédentes, dans lequel le moyen capteur comporte un indicateur de changement de couleur sensible au CO₂.

6. Un dispositif de confirmation de position de sonde d'alimentation tel que revendiqué dans la revendication 5, dans lequel l'indicateur de changement de couleur sensible au CO₂ est utilisable pour changer de couleur après positionnement de la portion prédéterminée de ladite sonde dans le système respiratoire du corps humain ou animal.

7. Un dispositif de confirmation de position de sonde d'alimentation tel que revendiqué dans n'importe lesquelles des revendications précédentes, dans lequel le moyen capteur comporte un indicateur de changement de couleur sensible au pH.

8. Un dispositif de confirmation de position de sonde d'alimentation tel que revendiqué dans la revendication 7, dans lequel l'indicateur de couleur de pH est utilisable pour changer en une couleur prédéterminée après positionnement d'une portion prédéterminée de ladite sonde dans l'estomac du corps humain ou animal.

9. Un dispositif de confirmation de position de sonde d'alimentation tel que revendiqué dans la revendication 7, dans lequel le changement en une couleur prédéterminée se produit à un pH inférieur à 6.

10. Un dispositif de confirmation de position de sonde d'alimentation tel que revendiqué dans les revendications 7 à 9, dans lequel l'indicateur de changement de couleur de pH est sélectionné parmi : rouge Congo ; bleu de bromophénol ; bleu de chlorophénol ; bleu de bromochlorophénol ; jaune de méthyle ; methyl-orange.

11. Un dispositif de confirmation de position de sonde d'alimentation tel que revendiqué dans n'importe lesquelles des revendications précédentes, comportant une pluralité de capteurs.

12. Un dispositif de confirmation de position de sonde d'alimentation tel que revendiqué dans n'importe lesquelles des revendications précédentes, suffisamment rigide pour être utilisable comme raidisseur afin de faciliter l'insertion de ladite sonde d'alimentation dans le corps humain ou animal.

13. Un dispositif de confirmation de position de sonde d'alimentation tel que revendiqué dans n'importe lesquelles des revendications précédentes comportant en outre un détecteur optique utilisable pour détecter la lumière de sortie et la convertir en un signal électronique indicatif de la position de la position prédéterminée de la sonde d'alimentation.

14. Un kit de sonde d'alimentation médicale comportant une sonde d'alimentation médicale et au moins un dispositif de confirmation de position de sonde d'alimentation tel que revendiqué dans les revendications 1 à 13.

15. Un kit de sonde d'alimentation médicale tel que revendiqué dans la revendication 14, comportant en outre une source de lumière.
